(19) Europäisches Patentamt

European Patent Office

Office européen des brevets

(11) Veröffentlichungsnummer: **0 289 921 B1**

## (12) EUROPÄISCHE PATENTSCHRIFT

(45) Veröffentlichungstag der Patentschrift: **11.03.92**

(21) Anmeldenummer: **88106699.7**

(22) Anmeldetag: **27.04.88**

(51) Int. Cl.⁵: **C07C 29/74**, C07C 67/03, C07C 67/54, C07C 211/05, C07C 211/03, C07C 209/82, C07C 69/06, //C07C29/38, C07C31/22

(54) Verfahren zur Gewinnung von Trialkylaminen und Methylformiat bei der Herstellung von Trimethylolalkanen.

(30) Priorität: **06.05.87 DE 3715035**

(43) Veröffentlichungstag der Anmeldung:
**09.11.88 Patentblatt 88/45**

(45) Bekanntmachung des Hinweises auf die
Patenterteilung:
**11.03.92 Patentblatt 92/11**

(84) Benannte Vertragsstaaten:
**AT BE DE FR GB IT NL SE**

(56) Entgegenhaltungen:
**EP-A- 0 142 090**
**DE-A- 1 952 738**
**DE-A- 2 507 461**

(73) Patentinhaber: **BASF Aktiengesellschaft**
**Carl-Bosch-Strasse 38**
**W-6700 Ludwigshafen(DE)**

(72) Erfinder: **Merger, Franz, Dr.**
**Max-Slevogt-Strasse 25**
**W-6710 Frankenthal(DE)**
Erfinder: **Hettinger, Peter, Dr.**
**Schloss-Strasse 3**
**W-6803 Edingen-Neckarhausen(DE)**
Erfinder: **Hupfer, Leopold, Dr.**
**Waltershoehe 3**
**W-6701 Friedelsheim(DE)**
Erfinder: **Paetsch, Juergen, Dr.**
**Am Altenbach 30**
**W-6706 Wachenheim(DE)**
Erfinder: **Deck, Heribert**
**Josef-Huber-Strasse 3**
**W-6700 Ludwigshafen(DE)**
Erfinder: **Auer, Heinz**
**Albrecht-Duerer-Strasse 12**
**W-6823 Neulussheim(DE)**
Erfinder: **Brunner, Erwin, Dr.**
**Neuhausener Weg 1**
**W-6700 Ludwigshafen(DE)**

## Beschreibung

Die vorliegende Erfindung betrifft ein Verfahren zur Abtrennung, bzw. Gewinnung von Trialkylaminen und Methylformiat aus Reaktionsgemischen, die bei der Herstellung von Trimethylolalkanen durch Umsetzung von n-Alkanalen mit 2,2 bis 4,5 mol Formaldehyd in wäßriger Lösung in Gegenwart von 0,6 bis 3 mol Trialkylaminen, jeweils bezogen auf 1 mol Alkanal und anschließende Hydrierung anfallen.

Gemäß der DE-OS 33 40 791 lassen sich Trimethylolalkane in guter Ausbeute herstellen, wobei die bei der Reaktion eingesetzten Trialkylamine in Form der Trialkylammoniumformiate destillativ aus dem Reaktionsgemisch abgetrennt werden. Trimethylolalkane hoher Reinheit zu isolieren, die in allen Anwendungsbereichen qualitativ befriedigen, gelingt nach diesem Verfahren nur unter Destillationsbedingungen, die großtechnisch nicht einfach realisierbar sind.

Der Erfindung lag die Aufgabe zugrunde, das beschriebene, vorteilhafte Verfahren noch vorteilhafter und wirtschaftlicher zu gestalten, indem man die Einsatzstoffe Trialkylamin und den nicht zu Trimethylolalkan umgesetzten Formaldehyd direkt oder in Form von Wertprodukten zurückgewinnt.

Demgemäß wurde ein Verfahren zur Gewinnung von Trialkylaminen und Methylformiat aus Reaktionsgemischen, die bei der Herstellung von Trimethylolalkanen durch Umsetzung von n-Alkanalen mit 2,2 bis 4,5 mol Formaldehyd in wäßriger Lösung in Gegenwart von 0,6 bis 3 mol Trialkylamin, jeweils bezogen auf 1 mol Alkanal und anschließende Hydrierung anfallen, gefunden, das dadurch gekennzeichnet ist, daß man das rohe Umsetzungsgemisch

a) auf Temperaturen von 100 bis 200 °C erhitzt, wobei man das im Reaktionsgemisch vorhandene Wasser weitgehend, gegebenenfalls zusammen mit überschüssigem, freiem Trialkylamin, destillativ abtrennt und das in Form von Trialkylammoniumformiat vorliegende Trialkylamin freisetzt unter Bildung von Trimethylolalkanformiat, das Trialkylamin abdestilliert und das Trimethylolalkanformiat mit Methanol gegenenfalls in Gegenwart katalytischer Mengen an Alkali- oder Erdalkalialkoholaten zu Trimethylolalkan und Methylformiat umestert oder

b) weitgehend entwässert, gegebenenfalls überschüssiges, freies Trialkylamin dabei abtrennt, das in Form von Trialkylammoniumformiat vorliegende Trialkylamin freisetzt, indem man das verbleibende Gemisch mit Methanol versetzt und auf Temperaturen von 100 bis 200 °C unter Bildung von Methylformiat und Trialkylamin erhitzt

und die Reaktionsprodukte in an sich bekannter Weise isoliert.

Gemäß dem Stand der Technik wird der Formaldehyd zweckmäßigerweise als wäßrige Lösung, z.B. als 10 bis 50 gew.%ige Lösung eingesetzt, so daß der Wassergehalt des Reaktionsgemisches z.B. bei 50 bis 85, vorzugsweise 60 bis 80, insbesondere bei 65 bis 75 Gew.%, bezogen auf das Reaktionsgemisch liegt. Erfindungsgemäß wird das nach der Hydrierung anfallende Gemisch weitgehend entwässert.

Nach der Verfahrensvariante a) erfolgt eine nahezu vollständige destillative Abtrennung des Wassers während des Erhitzens des rohen Umsetzungsgemisches auf Temperaturen von 100 bis 200, insbesondere 120 bis 180 °C, wobei gleichzeitig gegebenenfalls überschüssiges ungebundenes Trialkylamin entfernt wird. Das in Form von Trialkylammoniumformiat vorliegende Trialkylamin wird in diesem Temperaturbereich bei Atmosphärendruck, höheren oder niedrigeren Drücken unter Bildung von Trimethylolalkanformiat freigesetzt und durch Destillation aus dem Umsetzungsgemisch entfernt. Das Trimethylolalkanformiat wird dann mit Methanol gegebenenfalls in Gegenwart von katalytischen Mengen an Alkali- oder Erdalkalialkoholat zu Trimethylolalkan und Methylformiat umgeestert.

Die Methanolmenge ist nicht besonders kritisch. So kann man vorteilhaft zur Erzielung vollständiger Umesterung pro Mol Trimethylolalkanformiat 1 bis 20, insbesondere 3 bis 15 mol Methanol verwenden. Höhere Überschüsse sind möglich, bringen aber keine Vorteile.

Vorteilhaft kann man die Umesterung in Gegenwart katalytischer Mengen an Alkali- oder Erdalkalialkoholaten wie Calcium- oder Magnesiumalkoholat sowie insbesondere Natrium- oder Kaliumalkoholat vornehmen. Man kann vorzugsweise Alkoholate niedermolekularer Alkohole, die insbesondere 1 bis 6 Kohlenstoffatome enthalten können, verwenden, darunter besonders bevorzugt das Alkoholat des für die Umesterung eingesetzten Alkohols, d.h. Methanolate.

Die Katalysatormenge kann vorteilhaft etwa 0,005 bis 0,05 mol Alkoholat pro Mol Trialkylammoniumformiat betragen.

Nach der Umesterung kann das Reaktionsgemisch durch eine organische oder anorganische Säure, z.B. Essigsäure oder Salzsäure neutralisiert werden oder man kann die Metallionen mit Hilfe eines Kationenaustauschers unter Freisetzung des Alkohols aus dem Reaktionsgemisch entfernen. Die Reaktionsprodukte werden dann in an sich bekannter Weise, z.B. destillativ, isoliert.

Nach einer bevorzugten Ausführungsform des Verfahrens, gemäß Anspruch 1b ist es möglich, das Trialkylammoniumformiat direkt ohne die Zwischenstufe des Trimethylolalkanformiats in Methylformiat und Trialkylamin zu überführen, indem man das nach der Hydrierung anfallende Umsetzungsgemisch nach weitgehender Abtrennung von Wasser und gegebenenfalls nicht umgesetztem Trialkylamin mit Methanol versetzt und auf Temperaturen von 100 bis 200°C, vorzugsweise 120 bis 180°C erhitzt. Vor der Umsetzung mit Methanol wird das Wassergehalt des Reaktionsgemisches auf mindestens ca. 5 bis 15, insbesondere 5 bis 10 Gew.%, bezogen auf das Gemisch zurückgedrängt. Die Entfernung des Wassers kann zweckmäßigerweise durch Destillation, vorteilhaft durch Vakuumdestillation nach den dafür üblichen Techniken erfolgen.

Um einen vollständigen Umsatz zu erzielen, wird man mindestens äquimolare Mengen Methanol, bezogen auf Trialkylammoniumformiat zusetzen. Vorteilhaft kann man 1,0 bis 20, insbesondere 3 bis 15 mol Methanol je Mol Ammoniumformiat verwenden. Höhere Mengen sind möglich, bringen aber keine Vorteile. Enthält das rohe Reaktionsgemisch nach der Hydrierung bereits Trimethylolalkanformiate, so werden diese Ester zu Methylformiat und Trimethylolalkan umgesetzt.

Die Umsetzung des rohen Reaktionsgemisches mit Methanol kann diskontinuierlich oder vorzugsweise kontinuierlich, bei erhöhtem Druck, bevorzugt bei Drucken von 10 bis 25 bar sowie Temperaturen von ca. 100 bis 200°C durchgeführt werden.

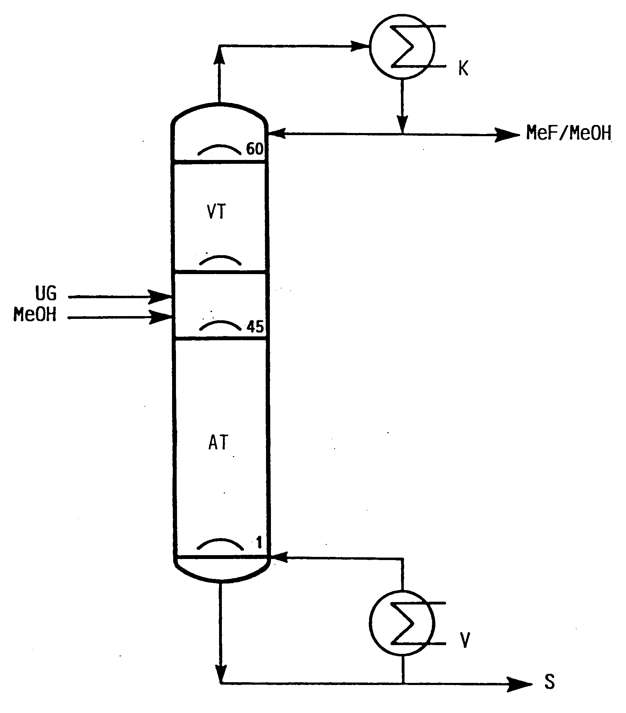

Vorzugsweise kann man eine kontinuierlich betriebene Reaktionskolonne, z.B. eine in Abbildung 1 dargestellte Glockenbodenkolonne, verwenden, die aus einem Abtriebsteil, AT, der eine ausreichende Verweilzeit der umzusetzenden Komponenten garantiert und einem Verstärkungsteil, VT, in welchem Methylformiat angereichert wird, aufgebaut ist.

Diese Glockenbodenkolonne beaufschlagt man zweckmäßigerweise auf einem mittleren Boden mit einem solchen Zulaufstrom aus Umsetzungsgemisch und Methanol, daß man im Bereich einer auf einen Boden bezogenen Belastung von 2 bis 100 g Umsetzungsgemisch pro ml Bodenvolumen und Stunde arbeitet. Je nach Belastung stellt man zur Herstellung eines spezifikationsgerechten Produktes ein Rücklaufverhältnis von 1:2 bis 1:100 ein.

Selbstverständlich kann man auch Reaktionskolonnen mit anderen Einbauten als Glockenböden benutzen.

Die im Zulauf der Reaktionskolonne, d.h. im rohen Umsetzungsgemisch, UG, das im wesentlichen aus Trimethylolalkan, Trialkylammoniumformiat, Wasser und zu geringen Anteilen Nebenprodukten wie Trimethylolalkanformiat besteht sowie im zugesetzten Methanol, MeOH, enthaltene Wassermenge sollte vorzugsweise 10 Vol.% der Gesamtzulaufmenge nicht übersteigen.

Das entstandene Methylformiat, MeF, wird im Gemisch mit Methanol am Kopf der Reaktionskolonne abgezogen und über einen Kühler, K, verflüssigt. Am Boden der Kolonne fallen freigesetztes Trialkylamin mit Ausnahme von Trimethylamin, das über Kopf abgetrennt wird sowie das bei der Reaktion entstandene bzw. im Zulauf enthaltene Wasser, nicht umgesetztes Methanol und Verbindungen, die schwerer flüchtig als Methylformiat sind, insbesondere das Trimethylolalkan sowie gegebenenfalls Nebenprodukte der Synthese an, die als Sumpfabzug, S, abgetrennt werden.

Die Aufarbeitung des Trialkylamin enthaltenden Sumpfprodukts erfolgt nach gängigen Trennverfahrenstechniken, z.B. destillativ, zur Gewinnung des Trialkylamins, des überschüssigen Methanols und der Trimethylolalkane.

Nach dem erfindungsgemäßen Verfahren kann ein vollständiger Umsatz des bei der Herstellung von Trimethylolalkanen im Reaktionsgemisch anfallenden Trialkylammoniumformiats sowie gegebenenfalls vorhandener Trimethylolalkanammoniumformiat zu Trialkylamin, Methylformiat und gegebenenfalls Trimethylolalkan erreicht werden, ohne daß die Ausbeuten bei der Trimethylolalkansynthese herabgesetzt werden.

Die Trimethylolalkansynthese erfolgt gemäß dem Stand der Technik, z.B. nach der DE-OS 33 40 791, so daß sich detaillierte Ausführungen hierzu erübrigen und nur einige grundsätzliche Bemerkungen gemacht werden sollen.

Als n-Alkanale werden beispielsweise Propanal, n-Butanal, n-Pentanal, 3-Methylbutanal, n-Hexanal, 3-Methylpentanal, n-Heptanal, 4-Methylhexanal, n-Octanal verwendet.

Als Trialkylamine kommen z.B. Trimethylamin, Triethylamin, Tri-n-propylamin oder Tri-n-butylamin in Betracht. Pro Mol des Alkanals können 2 bis 5 mol, vorzugsweise 2,5 bis 4 und insbesondere 2,75 bis 3,5 mol Formaldehyd und 0,6 bis 3 mol, vorzugsweise 0,7 bis 2 und insbesondere 0,8 bis 1,5 mol Trialkylamin eingesetzt werden.

Die Umsetzung wird im allgemeinen bei Temperaturen bis 120°C, vorzugsweise von 20 bis 200°C, insbesondere bei 40 bis 80°C, durchgeführt. Man arbeitet mit Unterdruck, Überdruck oder drucklos, vorzugsweise drucklos, diskontinuierlich oder kontinuierlich.

Die Reaktionszeiten liegen im allgemeinen bei etwa 0,5 bis 24 Stunden, häufig 1 bis 10, insbesondere 1 bis 7 Stunden.

Die Hydrierung des wäßrigen Reaktionsgemisches nimmt man auf an sich übliche Weise, zweckmäßigerweise bei Temperaturen von 80 bis 150°C und im Druckbereich von 20 bis 200 bar vor. Dabei kann man an sich übliche Hydrierkatalysatoren, wie solche, die Nickel, Kupfer oder Edelmetalle, wie Platin oder Palladium enthalten, verwenden. Die Katalysatoren können Trägerkatalysatoren sein und in der Suspensions- oder Festbettfahrweise angewendet werden. Besonders geeignete Katalysatoren der genannten Art werden z.B. in der DE-OS 30 27 890 beschrieben.

Das nach der Hydrierung anfallende Reaktionsgemisch wird in der erfindungsgemäßen Weise zur Gewinnung von Trialkylamin und Methylformiat umgesetzt.

Beispiel 1

In eine kontinuierlich betriebene Reaktionskaskade, die aus drei hintereinander geschalteten Kolben mit je 800 ml Reaktionsvolumen bestand, wurden stündlich 232 ml (1,67 mol) Triethylamin, 100,9 ml (1,15 mol) n-Butyraldehyd und 725,5 ml 15,2 %ige Formaldehydlösung (4 mol Formaldehyd) eingeleitet, wobei die Reaktionstemperatur in allen drei Kolben 80°C betrug. Der Kaskadenaustrag wurde einer kontinuierlichen Hydrierapparatur zugeführt und bei 115°C, 60 bar, hydriert (Katalysator: 81,47 % NiO; 18,53 % $Al_2O_3$). Anschließend wurden Wasser und überschüssiges Triethylamin durch Vakuumdestillation abgetrennt.

Der Destillationsrückstand, der einen Restwassergehalt von 5 % (Karl-Fischer-Methode) aufwies, wurde mit der gleichen Gewichtsmenge Methanol versetzt und mit einer Geschwindigkeit von 580 g/h auf den 45. Boden einer 60-bödigen bei 10 bar betriebenen Glockenbodenkolonne eingespeist.

Über Kopf der Kolonne wurde ein Methylformiat/Methanol-Gemisch der Zusammensetzung 63:37 entnommen, was einer Methylformiat-Ausbeute von 96 % bezüglich des in Formiat umgewandelten Formaldehyds entspricht. Der Sumpfabzug bestand aus ca. 39 Gew.% Methanol, 7 % Wasser, 14 % Triethylamin und 40 % eines Gemisches aus Trimethylolpropan, Di-trimethylolpropan und geringen Mengen Nebenprodukten.

Durch fraktionierende Destillation isolierte man daraus 120 g Trimethylolpropan/l Hydrieraustrag (Ausbeute 81 %, bezogen auf n-Butyraldehyd) vom $Sdp_3$ = 153°C sowie 22 g Di-trimethylolpropan/l Hydrieraustrag (Ausbeute 16 %, bezogen auf n-Butyraldehyd) vom Fp = 106 bis 108°C.

Beispiel 2

Zu 700 g 15 %iger Formalinlösung (3,5 mol Formaldehyd) und 150 g (1,5 mol) Triethylamin wurden innerhalb von 20 min 72 g (1 mol) n-Butyraldehyd getropft und die Mischung dann 5 h bei 73°C gerührt. Die homogene Lösung wurde bei 120°C, 50 bar einer kontinuierlichen Hydrierung zugeführt (Hydrierkontakt bestehend aus 81,47 % NiO; 18,53 % $Al_2O_3$) und der Reaktoraustrag bei Normaldruck durch Destillation vom Reaktionswasser und überschüssigem Triethylamin befreit.

Nach Anlegen eines Vakuums von 400 mbar erhitzte man den Destillationsrückstand bis zu einer Temperatur von 170°C, wobei Triethylamin entwich und durch Kondensation isoliert wurde. Nach ca. 5 h ging kein Amin mehr über; der Rückstand (142 g) wurde mit 100 g Methanol versetzt und nach Zugabe von 1,5 g Natriummethanolat 5 h lang bei Raumtemperatur gerührt. Anschließend wurde das Gemisch mit Essigsäure neutralisiert.

Durch Destillation bei Normaldruck trennte man zunächst ein Methylformiat/Methanol-Gemisch ab, das 51 g Methylformiat, entsprechend 91 % Ausbeute bezüglich des in Formiat umgewandelten Formaldehyds, enthielt.

Fraktionierende Destillation des Rückstands lieferte dann 108 g Trimethylolpropan vom $Kp_3$ = 153°C (≙ 81 % Ausbeute, bezogen auf n-Butyraldehyd) sowie 15 g Di-trimethylolpropan vom $Kp_3$ = 185°C (≙ 12 % Ausbeute, bezogen auf n-Butyraldehyd).

**Patentansprüche**

1. Verfahren zur Gewinnung von Trialkylaminen und Methylformiat aus Reaktionsgemischen, die bei der Herstellung von Trimethylolalkanen durch Umsetzung von n-Alkanalen mit 2,2 bis 4,5 mol Formaldehyd in wäßriger Lösung sowie 0,6 bis 3 mol Trialkylamin, jeweils bezogen auf 1 mol Alkanal und anschließende Hydrierung anfallen, dadurch gekennzeichnet, daß man das rohe Umsetzungsgemisch

   a) auf Temperaturen von 100 bis 200°C erhitzt, wobei man das im Reaktionsgemisch vorhandene Wasser weitgehend, gegebenenfalls zusammen mit überschüssigem, freiem Trialkylamin, destillativ abtrennt und das in Form von Trialkylammoniumformiat vorliegende Trialkylamin freisetzt unter Bildung von Trimethylolalkanformiat, das Trialkylamin abdestilliert und das Trimethylolalkanformiat mit Methanol gegebenenfalls in Gegenwart katalytischer Mengen an Alkali- oder Erdalkalialkoholaten zu Trimethylolalkan und Methylformiat umestert oder

   b) weitgehend entwässert, gegebenenfalls überschüssiges, freies Trialkylamin dabei abtrennt, das in Form von Trialkylammoniumformiat vorliegende Trialkylamin freisetzt, indem man das verbleibende Gemisch mit Methanol versetzt und auf Temperaturen von 100 bis 200°C unter Bildung von Methylformiat und Trialkylamin erhitzt

   und die Reaktionsprodukte in an sich bekannter Weise isoliert.

2. Verfahren nach Anspruch 1, dadurch gekennzeichnet, daß man dem Umsetzungsgemisch pro Mol Trimethylolalkanformiat 1 bis 20 mol Methanol zusetzt.

3. Verfahren nach Anspruch 1a, dadurch gekennzeichnet, daß man als Alkoholat Natrium- oder Kaliummethanolat verwendet.

4. Verfahren nach Anspruch 1a, dadurch gekennzeichnet, daß man 0,005 bis 0,05 mol Alkoholat je Mol Trimethylolalkanformiat verwendet.

5. Verfahren nach Anspruch 1a, dadurch gekennzeichnet, daß man das Trimethylolalkan bei Temperaturen von 0 bis 60°C mit Methanol umsetzt.

6. Verfahren nach Anspruch 1b, dadurch gekennzeichnet, daß man die Umsetzung mit Methanol kontinuierlich bei einem Druck von 10 bis 25 bar durchführt.

7. Verfahren nach Anspruch 1b, dadurch gekennzeichnet, daß man den Wassergehalt des Umsetzungsgemischs vor der Freisetzung des Trialkylamins auf mindestens ca. 5 bis 15 Gew.%, bezogen auf das Gemisch, herabsetzt.

**Claims**

1. A process for recovering trialkylamine and methyl formate from a reaction mixture obtained in the preparation of a trimethylolalkane by reaction of an n-alkanal with from 2.2 to 4.5 moles of formaldehyde in aqueous solution in the presence of from 0.6 to 3 moles of a trialkylamine, both quantities based on 1 mole of alkanal, and subsequent hydrogenation, wherein the crude reaction mixture is
    a) heated to from 100 to 200°C, the water present in the reaction mixture is substantially separated off by distillation, together with any excess free trialkylamine, the trialkylamine present in the form of a trialkylammonium formate is freed to form a trimethylolalkane formate, the trialkylamine is distilled off and the trimethylolalkane formate is transesterified with methanol to trimethylolalkane and methyl formate in the presence or absence of a catalytic amount of an alkali metal alkoxide or alkaline earth metal alkoxide, or
    b) substantially dewatered, any excess free trialkylamine being removed at the same time, the trialkylamine present in the form of a trialkylammonium formate is separated off by admixing the remaining mixture with methanol and heating the admixture to from 100 to 200°C to form methyl formate and trialkylamine,
    and the reaction products are isolated in a conventional manner.

2. A process as claimed in claim 1, wherein the reaction mixture is admixed with from 1 to 20 moles of methanol per mole of trimethylolalkane formate.

3. A process as claimed in claim 1a, wherein the alkoxide used is sodium methoxide or potassium methoxide.

4. A process as claimed in claim 1a, wherein from 0.005 to 0.05 mole of alkoxide is used per mole of trimethylolalkane formate.

5. A process as claimed in claim 1a, wherein the trimethylolalkane is reacted with methanol at from 0 to 60°C.

6. A process as claimed in claim 1b, wherein the reaction with methanol is carried out continuously under a pressure of from 10 to 25 bar.

7. A process as claimed in claim 1b, wherein the water content of the reaction mixture before the trialkylamine is freed is reduced to not less than about 5 - 15 % by weight, based on the mixture.

**Revendications**

1. Procédé d'obtention de trialkylamines et de formiate de méthyle à partir de mélanges réactionnels qui se forment lors de la préparation de triméthylolalcanes par réaction de n-alcanals avec 2,2 à 4,5 moles de formaldéhyde en solution aqueuse ainsi qu'avec 0,6 à 3 moles de trialkylamine, à chaque fois pour 1 mole d'alcanal et hydrogénation ultérieure, caractérisé en ce que
    a) on chauffe le mélange réactionnel brut à des températures de 100 à 200°C, en éliminant par distillation de façon poussée l'eau présente dans le milieu réactionnel, éventuellement avec de la trialkylamine libre en exces, on libère la trialkylamine existant sous forme de formiate de trialkylammonium avec formation de formiate de triméthylolalcane, on chasse par distillation la trialkylamine et on alcoolise le formiate de triméthylolalcane avec du méthanol, éventuellement en présence de quantités catalytiques d'alcoolates de métaux alcalins ou de métaux alcalino-terreux, en triméthylolalcane et formiate de méthyle ou

b) on déshydrate de façon poussée, on sépare de plus éventuellement la trialkylamine libre en excès, on libère la trialkylamine qui se trouve sous forme de formiate de trialkylammonium, en ajoutant du méthanol au mélange restant et en chauffant à des températures de 100 à 200°C avec formation de formiate de méthyle et de trialkylamine,
et on isole les produits de réaction d'une manière connue en soi.

2. Procédé selon la revendication 1, caractérisé en ce qu'on ajoute au mélange réactionnel de 1 à 20 moles de méthanol par mole de formiate de triméthylolalcane.

3. Procédé selon la revendication 1a, caractérisé en ce qu'on utilise comme alcoolate le méthanolate de sodium ou de potassium.

4. Procédé selon la revendication 1a, caractérisé en ce qu'on utilise 0,005 à 0,05 mole d'alcoolate par mole de formiate de triméthylolalcane.

5. Procédé selon la revendication 1a, caractérisé en ce qu'on fait réagir le triméthylolalcane avec le méthanol à des températures de 0 à 60°C.

6. Procédé selon la revendication 1b, caractérisé en ce qu'on effectue la réaction avec le méthanol en continu sous une pression de 10 à 25 bar.

7. Procédé selon la revendication 1b, caractérisé en ce qu'on amène la teneur en eau du mélange réactionnel avant la libération de la trialkylamine à moins d'environ 5 a 15% en poids, par rapport au mélange.